# EUROPEAN PATENT SPECIFICATION

(11) **EP 1 857 108 B1**
(45) Date of publication and mention of the grant of the patent: **08.12.2010**
(21) Application number: 06712758.9
(22) Date of filing: 01.02.2006
(51) Int. Cl.: A61K 31/422, A61K 45/06, A61K 31/4245, A61K 31/52, A61K 31/522, A61P 31/22, A61P 43/00, C07D 413/12

(54) **PREVENTIVE OR THERAPEUTIC AGENT FOR HERPESVIRUS-RELATED DISEASE**
VORBEUGE- ODER HEILMITTEL FÜR KRANKHEITEN IN ZUSAMMENHANG MIT DEM HERPES-VIRUS
AGENT PREVENTIF OU THERAPEUTIQUE CONTRE UNE MALADIE LIEE AU VIRUS DE L'HERPES

(30) Priority: 02.02.2005 JP 2005027107
(43) Date of publication of application: 21.11.2007
(73) Proprietor: Astellas Pharma Inc., Tokyo 103-8411 (JP)
(72) Inventor: SUZUKI, Hiroshi, 2-chome, Chuo-ku Tokyo, 1038411 (JP); SUDO, Kenji, 2-chome, Chuo-ku Tokyo, 1038411 (JP)
(74) Representative: Bates, Philip Ian
(86) International application number: PCT/JP2006/301615
(87) International publication number: WO 2006/082821

(56) References cited:
- EP-A- 1 340 750
- WO-A-2005/014559
- WO-A1-03/095435
- WO-A1-2005/014559
- DE-A1- 10 129 717
- DE-A1- 10 129 717
- CRUTE J J ET AL: "Herpes simplex virus helicase-primase inhibitors are active in animal models of human disease" NATURE MEDICINE, NATURE PUBLISHING GROUP, NEW YORK, NY, US, vol. 8, no. 4, 1 April 2002 (2002-04-01), pages 386-391, XP002969973 ISSN: 1078-8956

## Description

### Technical Field

This invention relates to a medicament, particularly a medicament useful for preventing and treating diseases associated with herpesvirus.

### Background Art

Viruses belonging to the Herpesviridae family cause various infectious diseases in human and animals. For example, it is known that varicella zoster virus (VZV) causes varicella and herpes zoster, and herpes simplex viruses types 1 and 2 (HSV-1 and HSV-2) cause infections such as herpes labialis, genital herpes, etc., respectively. In recent years, additionally, infectious diseases caused by herpesviruses such as cytomegalovirus (CMV), EB virus (Epstein-Barr virus; EBV), human herpesviruses 6, 7 and 8, etc. have been elucidated.
Currently, nucleic acid-based medicaments, so-called "polymerase inhibitor", such as acyclovir (ACV) and its prodrugs, i.e., valacyclovir (VCV), famciclovir (FCV), etc., are used as agents against herpesviruses such as VZV and HSV. These medicaments of nucleic acid series are mono-phosphorylated into nucleoside monophosphates by viral thymidine kinase encoded by VZV or HSV and are subsequently converted into triphosphate compounds by cellular enzymes. Finally, the tri-phosphorylated nucleoside analogues are incorporated during the replication of the viral genomes by herpesvirus DNA polymerase, to suppress the extension reaction of the viral DNA chains. Since the reaction mechanism of the existing anti-herpesvirus agents is based on the effect of the "competitive inhibition" toward deoxynucleoside triphosphate, as described above, it is necessary to use these drugs at a high concentration for the exertion of their antiviral effects. Actually, these anti-herpesvirus medicaments of nucleic acid series are clinically administered at a dose as high as several hundreds in mg to several grams per day. Since these medicaments of nucleic acid series can incorporate into the genome DNA of a host via the host DNA polymerase, further, the mutagenicity thereof draws concerns.
On the other hand, lately, several pharmaceutical drugs of non-nucleic acid-based and with anti-herpesvirus activity have been reported. For example, there is disclosed an amide or sulfonamide derivative suppressing the HSV helicase-primase enzyme complex to show anti-HSV-1 activity and anti-CMV activity, as represented by the following Formula (G), wherein the N atom is substituted with a thiazolylphenylcarbamoylmethyl group or the like (e.g., see Patent Reference 1). 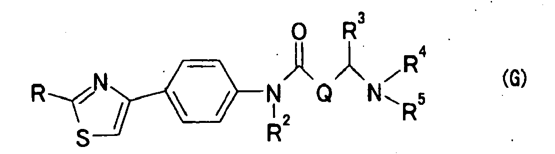 (In the formula, R is hydrogen, a lower alkyl, amino, lower alkylamino or the like; R² is hydrogen or a lower alkyl; Q may be absent or when it exists, Q represents a methylene; R³ is hydrogen, a lower alkyl or the like; R⁴ is an unsubstituted or substituted phenyl (lower) alkyl, 1-indanyl, 2-indanyl, (lower cycloalkyl)-(lower alkyl), (Het)-(lower alkyl) or the like; R⁵ is a phenylsulfonyl, 1-or 2-naphthylsulfonyl, (Het)-sulfonyl, (unsubstituted or substituted phenyl)-Y-(CH₂)ₙC(O), (Het)-(CH₂)ₙC(O) or the like, wherein Y is O or S and n is 0, 1 or 2; see the Patent Reference 1 for details.)
Further, there is disclosed an amide or sulfonamide derivative having anti-HSV-1 activity and anti-CMV activity as represented by the following Formula (H) wherein the nitrogen atom is substituted with a thiazolylphenylcarbamoylmethyl group (e.g., see Patent Reference 2). 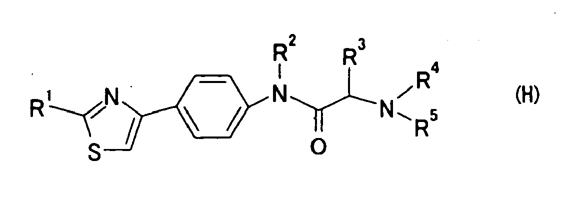 (In the formula, R¹ is NH₂; R² is H; R³ is H; R⁴ is CH₂Ph, CH₂-(4-pyridyl), CH₂-cyclohexyl or the like; and R⁵ is CO-(substituted phenyl), CO-(unsubstituted or substituted hetero ring) or the like; see the Publication for details.)
The present inventors previously found an amide compound substituted with a thiazolylphenylcarbamoylmethyl group and with favorable anti-herpesvirus activity, as represented by the following formula where the nitrogen atom of the amide group is substituted directly with an aromatic group aryl or heteroaryl group as ring A, or the salt thereof. Thus, the inventors filed a patent application (Patent Reference 3 and Patent Reference 4). 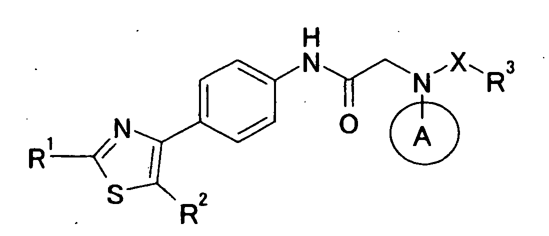 (In the formula, R¹ and R² represent -H, -lower alkyl, -NRaRb or the like; A represents -aryl which may have a substituent(s), -heteroaryl which may have a substituent(s) or the like; X represents CO or SO₂; R³ represents -aryl which may have a substituent(s), -heterocycle which may have a substituent(s) or the like; see the Publication for details).
In addition, a compound represented by the following formula is disclosed in an application by the instant applicant (Patent Reference 5) which was published after the priority date of the instant application. The compound is optionally used in a combination with acyclovir, valacyclovir or famciclovir. 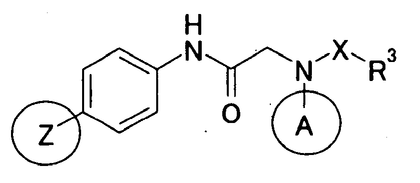 (In the formula, Z represents 1,2,4-oxadiazol-3-yl, 4-oxazolyl or the like, A represents an aryl group or the like which may have substituent(s), X represents CO or SO₂, and R³ represents a heterocycle or the like which may have substituent(s). See the Publication for detains.)
On the other hand, regarding compounds having a helicase-primase inhibitory activity but having a skeleton represented by the following formula which is different from the instant application, there are reports on the effect of their concomitant use with acyclovir (e.g., see Patent Reference 6). 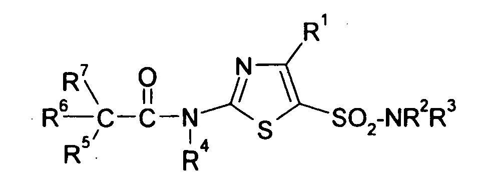 (See the Publication for the symbols in the formula.)

Patent Reference 1: International Publication No. 97/24343
Patent Reference 2: International Publication No. 00/29399
Patent Reference 3: International Publication No. 02/38554
Patent Reference 4: International Publication No. 03/95435
Patent Reference 5: International Publication No. 05/014559
Patent Reference 6: German Patent Application Publication No. 10129717 Specification

### Disclosure of the Invention

### Problems that the Invention is to Solve

To this day, development of an anti-herpesvirus agent having high safety is in great demand.

### Means for Solving the Problems

The present inventors have conducted extensive studies on agents for preventing and treating diseases associated with herpesvirus and, as a result, found two or more compounds having an anti-herpesvirus activity based on the helicase-primase inhibitory activity. In addition, helicase-primase inhibitior was separately used with a polymerase inhibitor generally used as an anti-herpesvirus agent.

That is, the present invention relates to
(1) an anti-herpesvirus agent characterized by combining a helicase-primase inhibitor, N-{2-[(4-substituted phenyl)amino]-2-oxoethyl}tetrahydro-2H-thiopyran-4-carboxamide compound represented by the following general formula (I), with a polymerase inhibitor 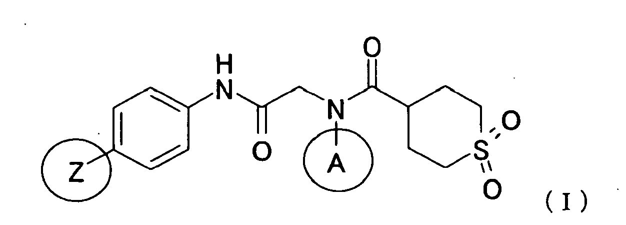 (symbols in the formula represent the following meanings Z: 1,2,4-oxadiazol-3-yl or 4-oxazolyl group, A: a phenyl group which is substituted by at least one methyl group and may further have 1 or 2 substituents selected from the group consisting of a methyl group and halogen atoms, or a 5-indanyl group, the same shall apply hereinafter), and
(2) the anti-herpesvirus agent described in the aforementioned (1), wherein the polymerase inhibitor is selected from acyclovir, valacyclovir and famciclovir; wherein the compound of general formula (I) and the polymerase inhibitor are for administration separately.
   Preferably, the polymerase inhibitor is valacyclovir.
In addition, it also relates to
(3) a medicament for treating herpesvirus infection, which comprises (a) a pharmaceutical preparation comprising the compound of general formula (I) as an active ingredient and (b) a package insert indicating that said pharmaceutical preparation is separately used with an anti-herpesvirus agent comprising a polymerase inhibitor as an active ingredient, wherein the polymerase inhibitor is selected from acyclovir, valacyclovir and famciclovir. Preferably, the polymerase inhibitor is valacyclovir.
Further, the invention also includes the following embodiments.
(4) A composition for treating herpesvirus infection comprising a compound of the general formula (I) and a polymerase inhibitor wherein the polymerase inhibitor is selected from acyclovir, valacyclovir and famciclovir; wherein the compound of general formula (I) and the polymerase inhibitor are for administration separately.
   Preferably, the polymerase inhibitor is valacyclovir.
(5) Use of the compound of general formula (I), for the manufacture of a medicament to be used for treating herpesvirus infection through the separate use with a polymerase inhibitor, wherein the polymerase inhibitor is selected from acyclovir, valacyclovir and famciclovir. Peferably, the polymerase inhibitor is valacyclovir.
(6) An agent for enhancing herpesvirus infection treating activity of a polymerase inhibitor, which comprises the compound of the general formula (I) as an active ingredient wherein the polymerase inhibitor is selected from acyclovir, valacyclovir and famciclovir; wherein the compound of general formula (I) and the polymerase inhibitor are for administration separately.
   Preferably, the polymerase inhibitor is valacyclovir.
(7) An agent for treating herpesvirus infection of a patient undergoing herpesvirus infection treatment with a polymerase inhibitor, which comprises the compound of the general formula (I) as an active ingredient wherein the polymerase inhibitor is selected from acyclovir, valacyclovir and famciclovir; wherein the compound of general formula (I) and the polymerase inhibitor are for administration separately.
   Preferably, the polymerase inhibitor is valacyclovir.
(8) An agent for treating herpesvirus infection, wherein the anti-herpesvirus activity is enhanced in comparison with the administration of a polymerase inhibitor alone, which comprises a combination of the polymerase inhibitor and the compound of the general formula (I) wherein the polymerase inhibitor is selected from acyclovir, valacyclovir and famciclovir; wherein the compound of general formula (I) and the polymerase inhibitor are for administration separately.
   Preferably, the polymerase inhibitor is valacyclovir.
(9) An agent for enhancing anti-herpesvirus activity for a patient administered with a polymerase inhibitor, which comprises an effective amount of the compound of the general formula (I) wherein the polymerase inhibitor is selected from acyclovir, valacyclovir and famciclovir; wherein the compound of general formula (I) and the polymerase inhibitor are for administration separately.
   Preferably, the polymerase inhibitor is valacyclovir.

### Effect of the Invention

By separate administration of a polymerase inhibitor acyclovir, valacyclovir or famciclovir as a conventional anti-herpesvirus agent with a helicase-primase inhibitor of general formula (I) having a different mechanism of action, the anti-herpesvirus agent of the present invention achieved an anti-herpesvirus activity. Therefore, it is particularly effective in a case in which a sufficient therapeutic effect cannot be achieved only with a polymerase inhibitor. In addition, since the doses of both agents can be kept low by their separate use, it is possible to obtain a therapeutic effect which is similar to or greater than the case of respective single administration, together with lowering the undesirable actions of both agents to be concerned. Accordingly, the anti-herpesvirus agent of the present invention is useful as an anti-herpesvirus agent particularly having high safety for the prevention or treatment of various herpesvirus infections such as varicella (chickenpox) associated with VZV infection, herpes zoster associated with recurrent infection with latent VZV, labial herpes and herpes encephalitis associated with HSV-1 infection, genital herpes associated with HSV-2 infection.

### Best Mode for Carrying Out the Invention

The helicase-primase inhibitor according to the present invention is an N-{2-[(4-substituted phenyl)amino]-2-oxoethyl}tetrahydro-2H-thiopyran-4-carboxamide compound represented by the following general formula (I). In this description, this may be simply referred sometimes as "helicase-primase inhibitor" hereinafter. 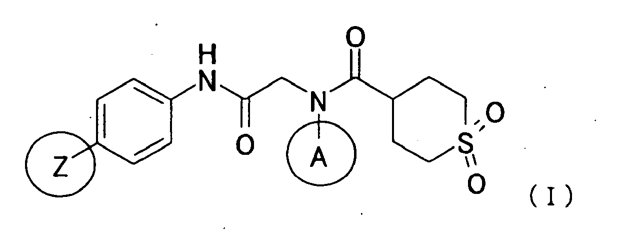 (Symbols in the formula have the following meanings Z: a 1,2,4-oxadiazol-3-yl or 4-oxazolyl group, A: a phenyl group which is substituted by at least one methyl group and may further have 1 or 2 substituents selected from the group consisting of a methyl group and halogen atoms, or a 5-indanyl group.)
Also, the polymerase inhibitor is a compound which inhibits the enzyme activity possessed by a DNA-polymerase complex of herpesvirus, and its illustrative examples include nucleic acid-based drugs such as acyclovir (ACV) and its prodrugs valacyclovir (VCV), famciclovir (FCV)
Particularly preferred is VCV.

The "anti-herpesvirus agent characterized by combining a helicase-primase inhibitor with a polymerase inhibitor" of the present invention includes a pharmaceutical preparation for preventing or treating a disease associated with herpesvirus, which comprises an effective amount of a helicase-primase inhibitor of general formula (I) and an effective amount of polymerase inhibitor such as acyclovir, valacyclovir or famciclovir (separate administration), and a kit which comprises two pharmaceutical preparations; an anti-herpesvirus agent as a first pharmaceutical preparation comprising a helicase-primase inhibitor of general formula (I) as its active ingredient and another anti-herpesvirus agent as a second pharmaceutical preparation comprising a polymerase inhibitor acyclovir, valacyclovir or famciclovir as its active ingredient. In this connection, the two pharmaceutical preparations are administered through the same or different route of administration separately.

The aforementioned "kit which comprises two pharmaceutical preparations" comprises two pharmaceutical preparations each of which contain respective active ingredients for separate use therapy of these active ingredients, and its example include a package which may contain a supplementary pharmaceutical preparation, such as placebo, which facilitates administration in response to their administration period on occasion demands or a display member. The "separately" means that the first pharmaceutical preparation and the second pharmaceutical preparation are separately administered through the same or different route of administration at the same or different administration frequency or administration interval. They are separately administered under suitable administration conditions for respective pharmaceutical preparations concerning pharmaceutical preparation formulation, route of administration, administration frequency, by taking bioavailability, stability of each pharmaceutical preparation into consideration.

In addition, the "medicine for anti-herpesvirus treatment, which comprises (a) a pharmaceutical preparation comprising a helicase-primase inhibitor of general formula (I) as an active ingredient and (b) a package insert indicating that said pharmaceutical preparation is separately used with an anti-herpesvirus treating agent comprising a polymerase inhibitor (acyclovir, valacyclovir or famciclovir) as the active ingredient" means a packaged medicine for anti-herpesvirus treatment, which comprises an effective amount of the pharmaceutical preparation comprising the helicase-primase inhibitor of general formula I as the active ingredient, shown by (a), and the package insert relating to the aforementioned pharmaceutical preparation (a) indicating that said pharmaceutical preparation is separately used with an anti-herpesvirus treating agent comprising a polymerase inhibitor (acyclovir, valacyclovir or famciclovir) as the active ingredient, shown by (b).

Next, the helicase-primase inhibitor of the medicine of the present invention, N-{2-[(4-substituted phenyl)amino]-2-oxoethyl}tetrahydro-2H-thiopyran-4-carboxamide compound represented by the general formula (I), is further described.
In the invention, F, Cl, Br and I atoms may be exemplified as a "halogen atom".
The N-{2-[(4-substituted phenyl)amino]-2-oxoethyl}tetrahydro-2H-thiopyran-4-carboxamide compound represented by the general formula (I) may be its hydrates, various species of solvates and polymorphic substances.

Particularly, the following compounds are desirable as the compounds represented by the general formula (I).
(1) A compound in which Z is a 1,2,4-oxadiazol-3-yl group.
(2) A compound in which Z is a 4-oxazolyl group.
(3) A compound in which A is a phenyl group which is substituted by at least one methyl group and may further have 1 or 2 substituents selected from the group consisting of a methyl group and halogen atoms.
(4) A compound in which A is a 5-indanyl group.
(5) A compound selected from the group consisting of
   N-(2,6-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(3,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,3-oxazol-4-yl)phenyl]amino}-2-oxoethyl)tetrahydro-2H-thiopyran-4-carboxamide 1,1-dioxide,
   N-(4-chloro-3-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-fluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(3-fluoro-2,4-dimethylphenyl)-N-(2-{[4-(1,3-oxazol-4-yl)phenyl]amino}-2-oxoethyl)tetrahydro-2H-thiopyran-4-carboxamide 1,1-dioxide,
   N-(3,5-difluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2-fluoro-4-methylphenyl)-N-(2-{[4-(1,3-oxazol-4-yl)phenyl]amino}-2-oxoethyl)tetrahydro-2H-thiopyran-4-carboxamide 1,1-dioxide,
   N-(2,3-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-fluoro-2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(2,3-dihydro-1H-inden-5-yl)-N-(2-{[4-(1,2,4-oxadiazol-3-yl)phenyl]amino}-2-oxoethyl)tetrahydro-2H-thiopyran-4-carboxamide 1,1-dioxide,
   N-(3-fluoro-4-methylphenyl)-N-(2-{[4-(1,2,4-oxadiazol-3-yl)phenyl]amino}-2-oxoethyl)tetrahydro-2H-thiopyran-4-carboxamide 1,1-dioxide,
   N-(4-chloro-3-methylphenyl)-N-(2-{[4-(1,2,4-oxadiazol-3-yl)phenyl]amino}-2-oxoethyl)tetrahydro-2H-thiopyran-4-carboxamide 1,1-dioxide, and
   N-(3-fluoro-2,4-dimethylphenyl)-N-(2-{[4-(1,2,4-oxadiazol-3-yl)phenyl]amino}-2-oxoethyl)tetrahydro-2H-thiopyran-4-carboxamide 1,1-dioxide.

The anti-herpesvirus agent of the present invention characterized by combining a helicase-primase inhibitor of general formula (I) with a polymerase inhibitor (acyclovir, valacyclovir or famciclovir) can be prepared as separate pharmaceutical preparations for a kit, from one or two or more species of effective amount of a helicase-primase inhibitor of general formula (I) and one or two or more species of effective amount of polymerase inhibitor acyclovir, valacyclovir or famciclovir, by a generally used method using carriers, excipients for medicaments generally used in this field. Administration thereof may be either oral via tablets, pills, capsules, granules, powders, liquids, or parenteral dosing via injections such as intravenous injections, intramuscular injections, external agents such as ointments, plasters, creams, jellies, cataplasm, sprays, lotions, eye drops, eye ointments, suppositories, inhalation agents.

As the solid composition for oral administration, tablets, powders, granules are used. In such a solid composition, one or more active substances are mixed with at least one inert excipient, for example, lactose, mannitol, glucose, hydroxypropyl cellulose, microcrystalline cellulose, starch, polyvinylpyrrolidone, magnesium metasilicate aluminate. According to general methods, the composition may contain inert additives such as lubricants, e.g., magnesium stearate, disintegrators, e.g., sodium carboxymethyl starch, and dissolution auxiliary agents. The tablets or pills may be coated with sugar coating or stomach-soluble or enteric coating.
Examples of the liquid composition for oral administration include pharmaceutically acceptable emulsions, liquids, suspensions, syrups, elixirs, in which inert solvents for general use such as purified water, ethanol, can be incorporated. In addition to the inert solvents, the composition may further contain auxiliary agents such as solubilizing agents, moistening agents and suspending agents; sweetening agents; flavoring agents; aromatic agents and preservatives.
Examples of the injections for parenteral administration include sterile aqueous or non-aqueous liquids, suspensions and emulsions. The aqueous solvents include, for example, distilled water for injections and physiological saline. The non-aqueous solvents include, for example, propylene glycol, polyethylene glycol, vegetable oils such as olive oil, alcohols such as ethanol, Polysorbate 80 (name in the pharmacopeia). Such compositions may further contain isotonic agents, antiseptics, moistening agents, emulsifying agents, dispersing agents, stabilizers and dissolution auxiliary agents. These are sterilized by filtering through bacteria-retaining filters, by incorporating sterilizing agents, or by irradiation. Alternatively, these may be produced into a sterile solid composition and then dissolved or suspended in sterile water or sterile solvents for injections prior to use.

Examples of the external agents include ointments, plasters, creams, jellies, cataplasms, sprays, lotions, eye drops, eye ointments. The external agent contains generally used ointment bases, lotion bases, aqueous or non-aqueous liquids, suspensions, emulsions. As the ointment or lotion bases, polyethylene glycol, propylene glycol, white Vaseline, white beeswax, polyoxyethylene hardened castor oil, glycerin monostearate, stearyl alcohol, cetyl alcohol, lauromacrogol, sorbitan sesquioleate, carboxyvinyl polymer, can be mentioned as examples.

When a compound conventionally known by the aforementioned reference is used as the helicase-primase inhibitor of the present invention, the appropriate dose and administration form described in said reference can be employed. Preferably, a dose smaller than the dose described in the reference is employed, because sufficient combined effect can be obtained with a further smaller amount. Generally, the suitable daily dose of the novel compound (I) of the present invention, which is the active ingredient of the present invention, is 0.001 to 50 mg/kg/body weight, preferably 0.01 to 30 mg/kg/body weight, more preferably 0.05 to 10 mg/kg/body weight, for oral administration. For intravenous administration, the daily dose is 0.0001 to 10 mg/kg/body weight, preferably 0.001 to 1.0 mg/kg/body weight. The dose is administered once or in separate portions per day, and is appropriately determined depending on each case, in terms of the symptom, age, sex. When the compound (I) is to be used as an external agent, the agent containing the compound of the present invention in an amount of 0.0001 to 20%, preferably 0.01 to 10%, is desirable. The external agent is administered locally once or in separate portions per day depending on the symptom.
On the other hand, dose of the polymerase inhibitor of the present invention is decided in response to the activity of the polymerase inhibitor to be used. In the case of a polymerase inhibitor in which its clinically suited dose and administration frequency are already known, it is desirable to administer it at said clinical dose and administration frequency. Alternatively, by taking its synergistic effect with the helicase-primase inhibitor into consideration, it may be administered at an amount smaller than that. For example, in the case of ACV, VCV or FCV, its daily dose is generally from 1 to 300 mg/kg, preferably from 5 to 200 mg/kg, more preferably from 10 to 150 mg/kg, per body weight in the case of oral administration, and its daily dose in the case of intravenous administration is from 0.01 to 10 mg/kg, preferably from 0.1 to 1.0 mg/kg, per body weight, respectively, and this is administered once a day or by dividing it into two or more times. The dose is optionally decided by taking the symptom, age, sex into consideration in response to individual cases. In addition, when used as an external preparation, an external preparation containing from 0.0001 to 20%, preferably from 0.01 to 10%, of the compound (I) is desirable. This is topically administered at from one to several times per day in response to the symptom.
The pharmaceutical composition of the present invention which comprises both of the helicase-primase inhibitor of general formula (I) and polymerase inhibitor acyclovir, valacyclovir or famciclovir is produced by preparing it in such a manner that both of the components are contained in respective amounts that correspond to the aforementioned doses.

The helicase-primase inhibitor of the present invention can be prepared by the method described in the aforementioned reference, and a commercially available product can be used as the polymerase inhibitor.
Hereinafter, typical production methods of the compound (I) as the helicase-primase inhibitor of the medicament of the present invention are described in the following. In this connection, the production methods are not limited to the following examples.
In the following production methods, it is sometimes effective from the viewpoint of the production technique to replace a certain functional group depending on the type with an appropriate protective group, namely a group readily convertible to the functional group, at the stage of a raw material or intermediate. Afterwards, the protective group can be eliminated, if necessary, to obtain the desired compound. Examples of such a functional group includes an amino group, hydroxyl group, carboxyl group and the like. Protective groups thereof are, for example, those described in Protective Groups in Organic Synthesis, the third edition (T. W. Green and P. G. M. Wuts, eds., JOHN WILLY & SONS, INC.). These may be appropriately used depending on the reaction conditions. For introducing and eliminating such protective groups, the methods described in the reference can be suitably applied.

### First Production Method

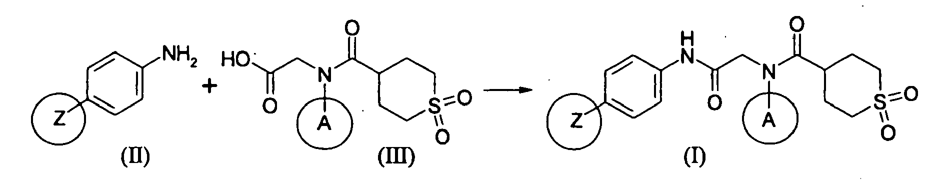

Compound (I) can be easily produced by subjecting Carboxylic Acid Compound (III) and Aniline Derivative (II) to an amidation reaction.
The amidation reaction can be carried out by general methods. For example, the method described in "Courses in Experimental Chemistry" edited by the Chemical Society of Japan, the fourth edition (Maruzen), Vol.22, pp.137-173 may be applicable. Preferably, the reaction is carried out by converting Carboxylic Acid Compound (III) to a reactive derivative such as an acid halide (acid chloride) or an acid anhydride, and then reacting the resulting reactive derivative with Aniline Derivative (II). In the case of using a reactive derivative of carboxylic acid, a base [an inorganic base such as potassium carbonate, sodium hydroxide, or an organic base such as triethylamine (TEA), diisopropylethylamine, pyridine] is preferably added. In addition, the amidation reaction may be carried out by reacting carboxylic acid in the presence of a condensation agent [1-ethyl-3-(3-dimethylaminopropyl)carbodiimide (WSC), 1,1'-carbonylbis-1H-imidazole (CDI)]. In this case, additives such as 1-hydroxybenzotriazole (HOBt), may be added. The reaction temperature can be appropriately selected depending on the raw material compound used. The solvent usable includes those inert to the reaction, for example, aromatic hydrocarbon-series solvents such as benzene, toluene; ether-series solvents such as tetrahydrofuran (THF), 1,4-dioxane; halogenated hydrocarbon-series solvents such as dichloromethane, chloroform; amide-series solvents such as N,N-dimethylformamide (DMF), N,N-dimethylacetamide, etc.; basic solvents such as pyridine. The solvent is appropriately selected depending on the type of the raw material compound, and can be used alone or as a mixture of two or more of them.

The aforementioned raw material compounds can be easily produced using known reactions, e.g., those described in "Courses in Experimental Chemistry" edited by the Chemical Society of Japan (Maruzen), in the pamphlet of the International Publication WO 02/38554. The typical production methods thereof are described below.

### Production method of Compound (III)

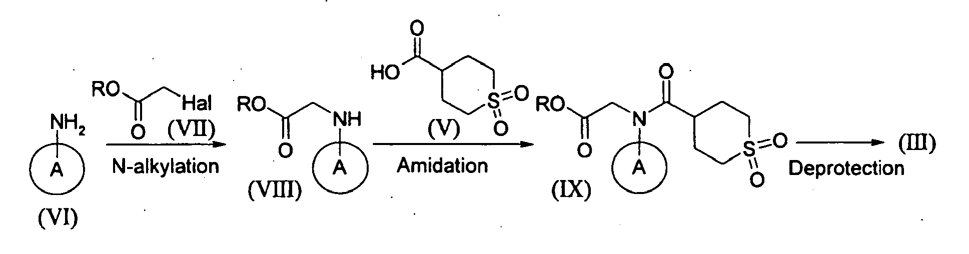

(In the formula, Hal means halogen, R means a group capable of forming an ester residue, such as a lower alkyl, an aralkyl)
In the reaction scheme above, amidation can be carried out in the same manner as in the first production method above.
N-alkylation of Compound (VI) can be carried out using Halogenated Alkyl Compound (VII) according to usual methods, e.g., the method described in the aforementioned "Courses in Experimental Chemistry", the fourth edition (Maruzen), Vol.20, pp.279-318. The reaction can be carried out under the temperature of from cooling to heating. Examples of the solvent usable include solvents inert to the reaction, for example, those exemplified for the amidation in the first production method. The reaction is carried out preferably in the presence of a base such as potassium carbonate, sodium hydroxide, sodium hydride. The amidation can be carried out in the same manner as in the first production method above. Herein, the amidation may be first carried out and subsequently, the N-alkylation may be carried out.

Deprotection for obtaining Carboxylic Acid Compound (III) can be carried out by appropriately applying a general method depending on the ester type. In the case of alkyl esters such as an ethyl ester, the deprotection can be preferably carried out by treating them with a base such as sodium hydroxide aqueous solution. In the case of aralkyl esters such as a benzyl ester, the deprotection can be carried out by reducing them with palladium-carbon (Pd-C) under hydrogen atmosphere. The reactions can be carried out according to the method described in the aforementioned "Protective Groups in Organic Synthesis", the third edition.
A desired raw material compound can be produced by subjecting the compound with a certain substituent type to a substituent modification reaction well known to those skilled in the art.
The compound (I) of the present invention obtained in this manner is isolated and purified in its free form or as a salt thereof after a salt formation process by a general method. The isolation and purification are carried out by employing general chemical procedures such as extraction, concentration, evaporation, crystallization, filtration, recrystallization, various chromatographic techniques.

### Reference examples

Effects as the medicament of the present invention were confirmed by the following pharmacological tests.

### Example 1 Helicase-primase inhibitory activity

Using an Baculoviruses for expressing respective proteins of UL5, UL52 and UL8 which constitute an HSV-1 helicase-primase complex (obtained from Dr. Nigel D. Stow, Medical Research Council, UK), a recombinant HSV-1 helicase-primase complex was prepared by the method described in a report of Crute et al. (J. B. C., 1991, Vol. 266, p. 21252 - 21256). Detection of DNA-dependent ATPase activity of the HSV-1 helicase-primase complex was carried out in reference to the method described in a report of Crute et al. (J. B. C., 1991, Vol. 266, p. 4484 - 4488). In brief, 520 ng of the HSV-1 helicase-primase complex was allowed to undergo the reaction at 30°C for 30 minutes in a reaction liquid containing 20 µg/ml of heat-denatured bovine sperm DNA and 2 mM ATP, and then concentration of phosphoric acid formed through the hydrolysis of ATP into ADP and monophosphate by the activity of ATPase was determined by adding the same volume of Malachite Green reagent (0.03% Malachite Green, 0.1% ammonium molybdate, 4.8% sulfuric acid) and measuring the resulting absorbance at 650 nm. The 50% inhibition concentration (IC₅₀ value) of each compound to be tested for HSV-1 helicase-primase DNA-dependent ATPase was calculated as the concentration which halves the phosphoric acid concentration at the time of not adding the compound. The results are shown in the following table.

**[Table 1]**

| Compounds to be tested | IC₅₀ value (µM) |
|---|---|
| Compound of Preparation 2 | 0.084 |
| Compound of Preparation 27 | 0.11 |

### Example 2 HSV-1 skin infection mouse model (in vivo test)

Using a cutaneous HSV-1 infection mouse model prepared in accordance with the method of H. Machida et al. (Antiviral Res., 1992, 17, 133 - 143), in vivo activity of the pharmaceutical composition of the present invention was tested. The skin of each HR-1 hairless mouse [female, 7 weeks of age] was scratched lengthwise and breadthwise several times using a needle and a virus suspension (HSV-1 strain WT-51, 1.5 x 10⁴ PFU/15 µl) was dropped to the scarified region for infection, while anesthetized with diethyl ether.
Regarding the compounds to be tested, VCV was used as the polymerase inhibitor and made into a methyl cellulose suspension, the compound of Preparation 2, N-(4-methylphenyl)-N-(2-{[4-(1,3-oxazol-4-yl)phenyl]amino}-2-oxoethyl)tetrahydro-2H-thiopyran-4-carboxamide 1,1-dioxide, which is described later, was used as the helicase-primase inhibitor and made into a methyl cellulose suspension, and they were orally administered at the doses of the following table twice a day for 5 days starting after 3 hours of the infection. The symptom of the skin lesion caused by HSV-1 infection were classified in the following scores for 17 days:
Score 0: no signs of infection.
Score 1: localized, barely perceptible small vesicles.
Score 2: slight vesicle spread.
Score 3: large patches of vesicles formed.
Score 4: zosteriform vesicles.
Score 5: large patches of ulcers formed.
Score 6: zosteriform with severe large ulcers.
Score 7: hind limb paralysis or death.
The AUC value was calculated from each group's mean disease score, and the disease inhibitory rate of the group administered with each test compound to the placebo group was calculated using the AUC. The results are shown in Table below. The concomitant use group of VCV with the compound of Preparation 2 showed good lesion inhibitory activity in comparison with the VCV single administration group, and the concomitant use group of 30 mg/kg of VCV with 3 mg/kg of the compound of Preparation 2 almost completely inhibited the lesions.

**[Table 2]**

| | VCV dose (mg/kg) | Dose of the compound of Preparation 2 (mg/kg) | Lesion inhibitory ratio (%) |
|---|---|---|---|
| Comparative Example 1 | 0 | 0 | 0 |
| Comparative Example 2 | 0 | 1 | 38 |
| Comparative Example 3 | 0 | 3 | 52 |
| Comparative Example 4 | 10 | 0 | 45 |
| Comparative Example 5 | 30 | 0 | 57 |
| Concomitant use 1 of the present invention | 10 | 1 | 76 |
| Concomitant use 2 of the present invention | 10 | 3 | 83 |
| Concomitant use 3 of the present invention | 30 | 1 | 86 |
| Concomitant use 4 of the present invention | 30 | 3 | 91 |

In the same manner as described in the above, VCV was used as the polymerase inhibitor and the compound of Preparation 27, N-(2,6-dimethylphenyl)-N-(2-{[4-(1,2,4-oxadiazol-3-yl)phenyl]amino}-2-oxoethyl)tetrahydro-2H-thiopyran-4-carboxamide 1,1-dioxide, which is described later, was used as the helicase-primase inhibitor, with the results shown in the following table.

**[Table 3]**

| | VCV dose (mg/kg) | Dose of the compound of Preparation 27 (mg/kg) | Lesion inhibitory ratio (%) |
|---|---|---|---|
| Comparative Example 6 | 0 | 0 | 0 |
| Comparative Example 7 | 0 | 1 | 33 |
| Comparative Example 8 | 0 | 3 | 68 |
| Comparative Example 9 | 10 | 0 | 42 |
| Comparative Example 10 | 30 | 0 | 52 |
| Concomitant use 5 of the present invention | 10 | 1 | 66 |
| Concomitant use 6 of the present invention | 10 | 3 | 86 |
| Concomitant use 7 of the present invention | 30 | 1 | 88 |
| Concomitant use 8 of the present invention | 30 | 3 | 100 |

The concomitant use group of 10 mg/kg of VCV with 3 mg/kg of the compound of Preparation 27, the concomitant use group of 30 mg/kg of VCV with 1 mg/kg of the compound of Preparation 27 and the concomitant use group of 30 mg/kg of VCV with 3 mg/kg of the compound of Preparation 27 showed good lesion inhibitory activity in comparison with the VCV single administration group, and the concomitant use group of 30 mg/kg of VCV with 3 mg/kg of the compound of Preparation 27 completely inhibited the lesions.

As in the above, it was confirmed in the *in vivo* animal model that the anti-herpesvirus activity was reinforced by the concomitant use of a VCV or the like polymerase inhibitor with a helicase-primase inhibitor, and more strong effect would be expected even at the same conventional polymerase inhibitor dose, or the same or further superior effect would be expected at a polymerase inhibitor dose of smaller than the conventional case. Even in a case in which a sufficient activity cannot be obtained by one mechanism, good anti-herpesvirus activity can be attained by the concomitant use of both agents, based on their synergistic effect by two mechanisms, so that excellent clinical effects can be obtained.

### (Preparations)

Production examples of the compound (I), which is an active ingredient of the present invention, are shown below as Preparations. Herein, many of the raw material compounds for use in the following reactions are known in the pamphlet of the Patent Reference 1 (International Publication WO 02/38554), and can therefore be readily available according to the methods described in these known references. Production examples of novel compounds among the raw materials are shown below in Reference Examples.

### Reference Example 1:

5% Palladium-carbon powder was added to an ethanol-tetrahydrofuran mixed suspension of 4-(4-nitrophenyl)-1,3-oxazol and stirred for 12 hours at room temperature in a hydrogen atmosphere. The reaction solution was filtered through Celite and the filtrate was evaporated under reduced pressure. The resulting crude product is purified with a silica gel column chromatography to obtain [4-(1,3-oxazol-4-yl)phenyl]amine (pale yellow solid). Electron Impact-MS(M)⁺. 160.

### Reference Example 2:

Potassium carboxylate and ethyl bromoacetate were added to a DMF solution of 4-methylaniline and heated while stirring. The reaction mixture was added with water and ethyl acetate. After the organic layer was separated, washed and dried, the solvent was evaporated under reduced pressure to obtain a crude product. The crude product was dissolved in methylene chloride, and pyridine, tetrahydro-2H-thiopyrane-4-carbonyl chloride 1,1-dioxide were added to the resulting solution and stirred. After the reaction solution was concentrated, 1M hydrochloric acid and chloroform were added. The organic layer separated was washed and dried and the solvent was evaporated under reduced pressure. The resulting crude product was purified with a silica gel column chromatography to obtain ethyl {[(1,1-dioxotetrahydro-2H-thiopyran-4-yl)carbonyl](4-methylphenyl)amino}acetate (colorless oily product). FAB-MS [(M+H)⁺] : 354.

### Reference Examples 3 to 15:

Compounds of Reference Examples 3 to 15, which are described in Table 4 below, were obtained in the same manner as in Reference Example 2.

### Preparation 1:

To an ethanol (10 ml) solution of ethyl {(2,6-dimethylphenyl)[(1,1-dioxide tetrahydro-2H-thiopyran-4-yl)carbonyl]amino}acetate (735 mg) was added aqueous 1M sodium hydroxide solution (2.3 mL). The mixture was stirred at room temperature for 5 hours. After 1M hydrochloric acid was added to the reaction mixture to make the solution acidic, water and chloroform were added thereto to separate the organic layer. Further, the organic layer was dried over anhydrous sodium sulfate and filtered, and then, the solvent was evaporated under reduced pressure. After the resulting crude carboxylic acid product was dissolved in chloroform (15 ml), WSC·HCl (422 mg) and [4-(1,3-oxazol-4-yl)phenyl]amine (320 mg) were added sequentially to the resulting solution, which was stirred at room temperature for 4 hours. After a saturated sodium hydrogencarbonate aqueous solution and chloroform were added to the reaction solution, the organic layer was separated. The organic layer was washed with a saturated sodium chloride solution, dried over anhydrous magnesium sulfate and filtered, from which the solvent was evaporated under reduced pressure. The resulting crude product was rinsed in hexane-ethyl acetate (= 3/2), and then recrystallized from ethanol, to obtain N-(2,6-dimethylphenyl)-N-(2-{[4-(1,3-oxazol-4-yl)phenyl]amino}-2-oxoethyl)tetrahydro-2H-thiopyran-4-carboxamide 1,1-dioxide (colorless crystal) in a yield of 610 mg.

### Preparations 2 - 40:

Compounds of Preparations 2 to 40 shown in Tables 5 to 12 below were obtained in the same manner as in Preparation 1.
The physicochemical properties of the compounds of Reference Examples are shown in Table 4, while Tables 5 to 12 show the structures and physicochemical properties of the compounds of Preparations.

Abbreviations in the tables indicate as follows. Ref: Reference Example; Ex: Preparation; Dat: physico-chemical properties {F+: FAB-MS [(M+H)⁺]; F-: FAB-MS [(M-H)⁻]; N1: ¹H-NMR (DMSO-d₆, TMS internal standard); mp: melting point (°C), solvent for crystallization is shown in the parentheses}; Ph: phenyl; Me: methyl; Et: ethyl; and iPr: isopropyl. Herein, the numerical figure before each substituent indicates the position for its substitution. For example, 3,4-(Cl)₂-5-F-Ph indicates a 3,4-dichloro-5-fluorophenyl group.

**[Table 4]**

| | | | | | | | |
|---|---|---|---|---|---|---|---|
| 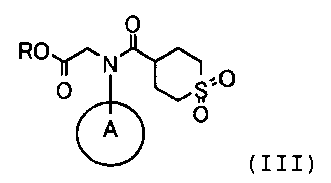 | | | | | | | |

| Ref | A | R | Dat | Re f | A | R | Dat |
|---|---|---|---|---|---|---|---|
| 3 | 2,3-(Me)₂-Ph | Et | F+: 368 | 4 | 4-Me-Ph | Et | F+: 354 |
| 5 | 2,5-(Me)₂-Ph | Et | F+: 368 | 6 | 3-Me-Ph | Et | F+: 354 |
| 7 | 3,4-(Me)₂-Ph | Et | F+: 368 | 8 | 2-Me-Ph | Et | F+: 354 |
| 9 | 2,4,6-(Me) ₃-Ph | Et | F+: 382 | 10 | 2,4-(Me)₂-Ph | Et | F+: 368 |
| 11 | 4-F-3-Me-Ph | Et | F+: 372 | 12 | 2,6-(Me)₂-Ph | Et | F+: 368 |
| 13 | 3-Br-4-Me-Ph | Et | F+: 432, 434 | 14 | 4-F-2,6-(Me)₂-Ph | Et | F+: 386 |
| 15 | 3,5-(Me)₂-Ph | Et | F+: 368 | | | | |

**[Table 5]**

| | | |
|---|---|---|
| 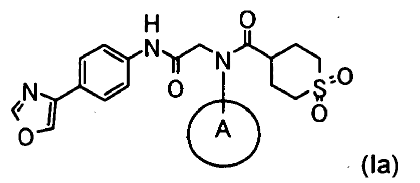 | | |

| **Ex** | **A** | **Dat** |
|---|---|---|
| | | F+: 482 |
| 1 | 2,6-(Me)₂-Ph | N1: 1.87-2.42(5H, m), 2.13(6HX0.1, s), 2.33(6HX0.9, s), 2.97-3.27(4H, m), 4.19(2HX0.9, s), 4.48(2HX0.1, s), 7.0 7-7.25(3H, m), 7.62-7.66(2H, m), 7.72-7.75(2H, m), 8.43 (1H, d), 8.54(1H, d), 10.15(1H, brs) |
| | | mp: 224-227 (EtOH) |
| | | F+: 468 |
| 2 | 4-Me-Ph | N1: 1.98-2.06(4H, m), 2.34(3H, s), 2.68-2.70(1H, m), 2.9 7-3.02(4H, m), 4.35(2H, s), 7.28 (2H, d), 7.36(2H, d), 7 .63-7.66(2H, m), 7.72-7.76(2H, m), 8.43(1H, s), 8.54(1H , s), 10.14(1H, s) |
| | | mp: 199-201 (EtOH-H₂O) |
| | | F+: 468 |
| 3 | 3-Me-Ph | N1: 2.01-2.09(4H, m), 2.35(3H, s), 2.71(1H, m), 2.93-3.06 (4H, m), 4.36(2H, s), 7.17-7.38 (4H, m), 7.64(2H, d), 7. 73(2H, d), 8.43(1H, d), 8.54(1H, d), 10.15(1H, s) |
| | | F+: 468 |
| 4 | 2-Me-Ph | N1: 1.88-2.15(4H, m), 2.15(3HX0.1, s), 2.26(3HX0.9, s), 2.41-2.46(1H, m), 2.83-3.05(4H, m), 3.86(1HX0.9, d), 4. 20(1HX0.1, d), 4.74(1HX0.9, d), 4.84(1HX0.1, d), 7.09-7 .77(8H, m), 8.43(1H, d), 8.53(1H, d), 10.14(1HX0.9, s), 10.19(1HX0.1, s) |
| | | mp: 220-221 (EtOH) |

**[Table 6]**

| | | |
|---|---|---|
| | | F+: 482 |
| 5 | 2,3-(Me)₂-Ph | N1: 1.85-2.12(4H, m), 2.03(3HX0.1, s), 2.15(3HX0.9, s), 2.25(3HX0.1, s), 2.31(3HX0.9, s), 2.42-2.47(1H, m), 2.8 3-2.90(1H, m), 3.00-3.22(3H, m), 3.84(1HX0.9, d), 4.16( 1HX0.1, d), 4.72(1HX0.9, d), 4.84(1HX0.1, d), 7.07-7.36 (3H, m), 7.62-7.66(2H, m), 7.71-7.76(2H, m), 8.43(1H, brs), 8.54(1H, d), 10.12(1HX0.9, s), 10.16(1HX0.1, s) |
| | | mp: 185-187 (EtOH) |
| | | F+: 482 |
| 6 | 2,4-(Me)₂-Ph | N1: 1.88-2.50(5H, m), 2.09(3HX0.1, s), 2.21(3HX0.9, s), 2.25(3HX0.1, s), 2.30(3HX0.9, s), 2.85-3.20(4H, m), 3.8 1(1HX0.9, d), 4.17(1HX0.1, d), 4.72(1HX0.9, d), 4.81(1 HX0.1, d), 6.97-7.39(3H, m), 7.62-7.66(2H, m), 7.72-7.7 6(2H, m), 8.43(1H, s), 8.54(1H, s), 10.11(1HX0.9, s), 1 0.17(1HX0.1, s) |
| | | mp: 176-177 (EtOH) |
| | | F+: 482 |
| 7 | 2,5-(Me)₂-Ph | N1: 1.86-2.51(5H, m), 2.08(3HX0.1, s), 2.20(3HX0.9, s), 2.22(3HX0.1, s), 2.30(3HX0.9, s), 2.87-3.26(4H, m), 3.8 4(1HX0.9, d), 4.21(1HX0.1, d), 4.70(1HX0.9, d), 4.80(1 HX0.1, d), 6.92-7.32(3H, m), 7.63-7.65(2H, m), 7.72-7.7 6(2H, m), 8.43(1H, s), 8.54(1H, s), 10.12(1HX0.9, s), 1 0.17(1HX0.1, s) |
| | | mp: 201-202 (EtOH) |
| | | F+: 482 |
| 8 | 3,4-(Me)₂-Ph | N1: 1.92-2.08(4H, m), 2.09(3H, s), 2.24(3H, s), 2.71(1H, s), 2.94-3.06(4H, m), 4.33(2H, S), 7.17-7.24(3H, m), 7.6 4(2H, d), 7.73(2H, d), 8.43(1H, s), 8.54(1H, s), 10.12(1 H, s) |
| | | F+: 482 |
| 9 | 3,5-(Me)₂-Ph | N1: 1.96-2.14(4H, m), 2.30(6H, s), 2.73(1H, m), 2.95-3.04 (4H, m), 4.33(2H, S), 7.02(1H, s), 7.08(2H, s), 7.64(2H, d), 7.73(2H, d), 8.43(1H, s), 8.54(1H, s), 10.12(1H, s) |
| | | mp: 205-206 (EtOH) |

**[Table 7]**

| | | |
|---|---|---|
| | | F+: 496 |
| 10 | 2,4,6-(Me) ₃-Ph | N1: 1.87-2.45(5H, m), 2.08(3HX0.1, s), 2.09(6HX0.1, s), 2.27(3HX0.9, s), 2.28(6HX0.9, s), 3.01-3.26(4H, m), 4.1 6.(2HX0.9, s), 4.44(2HX0.1, s), 6.88(2HX0.1, s), 7.01(2H X0.9, s), 7.61-7.65(2H, m), 7.71-7.75(2H, m), 8.43(1H, s), 8.54(1H, s), 10.12(1HX0.9, s), 10.14(1HX0.1, s) |
| | | mp: 237-238 (EtOH) |
| | | F+: 494 |
| 11 | 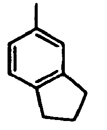 | N1: 2.01-2.08(6H, m), 2.70-3.06(9H, m), 4.34(2H, s), 7.1 3-7.32(3H, m), 7.64(2H, d), 7.73(2H, d), 8.43(1H, s), 8. 54(1H, s), 10.13(1H, s) |
| | | F+: 502 |
| 12 | 3-Cl-4-Me-Ph | N1: 2.01-2.06(4H, m), 2.36(3H, s), 2.68-2.75(1H, m), 3.0 1-3.06(4H, m), 4.37(2H, S), 7.37-7.40(1H, m), 7.46(1H, d), 7.60-7.66(3H, m), 7.74(2H, d), 8.44(1H, s), 8.55(1H, s), 10.18(1H, s) |
| | | mp: 146-148 (EtOH) |
| | | F+: 502 |
| 13 | 4-Cl-3-Me-Ph | N1: 2.00-2.06(4H, m), 2.36(3H, s), 2.68-2.75(1H, m), 3.0 1-3.04(4H, m), 4.36(2H, S), 7.33-7.36(1H, m), 7.48-7.52 (2H, m), 7.64(2H, d), 7.73(2H, d), 8.43(1H, s), 8.54(1H, s), 10.18(1H, s) |
| | | mp: 133-135 (EtOH) |
| | | F+: 486 |
| 14 | 3-F-4-Me-Ph | N1: 2.00-2.05(4H, m), 2.26(3H, s), 2.70-2.77(1H, m), 3.0 1-3.03(4H, m), 4.36(2H, S), 7.24-7.26(1H, m), 7.32-7.41 (2H, m), 7.64(2H, d), 7.73(2H, d), 8.43(1H, s), 8.54(1H, s), 10.17(1H, s) |
| | | mp: 135-137 (EtOH) |
| | | F+: 546, 548 |
| 15 | 3-Br-4-Me-Ph | N1: 2.00-2.06(4H, m), 2.38(3H, s), 2.68-2.74(1H, m), 3.0 1-3.04(4H, m), 4.36(2H, S), 7.41-7.47(2H, m), 7.64(2H, d), 7.73-7.76(3H, d), 8.43(1H, s), 8.54(1H, s), 10.18(1H, s) |
| | | mp: 154-155 (EtOH) |

**[Table 8]**

| | | |
|---|---|---|
| | | F+: 486 |
| 16 | 5-F-2-Me-Ph | N1: 1.88-2.15(4H, m), 2.11(3HX0.1, s), 2.23(3HX0.9, s), 2.45-2.49(1H, m), 2.96-3.16(4H, m), 3.92(1HX0.9, d), 4. 27(1HX0.1, d), 4.70(1HX0.9, d), 4.82(1HX0.1, d), 6.95-6 .98(1HX0.1, m), 7.06-7.10(1HX0.1, m), 7.20-7.25(1HX0.9 , m), 7.29-7.33(1HX0.1, m), 7.37-7.7.40(1HX0.9, m), 7.4 2-7.46(1HX0.9, m), 7.65(2H, d), 7.74(2H, d), 8.43(1H, s ), 8.54(1H, s), 10.18(1HX0.9, s), 10.23(1HX0.1, s) |
| | | mp: 235-236 (EtOH) |
| | | F+: 500 |
| 17 | 3-F-2,4-(Me)₂-Ph | N1: 1.88-2.23(4H, m), 2.03(3HX0.1, s), 2.16(3HX0.9, s), 2.20(3HX0.1, s), 2.26(3HX0.9, s), 2.47-2.54(1H, m), 2.8 7-3.17(4H, m), 3.91(1HX0.9, d), 4.25(1HX0.1, d), 4.66(1 HX0.9, d), 4.80(1HX0.1, d), 6.88 (1HX0.1, d), 7.10(1HX 0.1, dd), 7.21 (1HX0.9, dd), 7.28(1HX0.9, d), 7.64(2H, d), 7.73(2H, s), 8.43(1H, s), 8.54(1H, s), 10.14(1HX0.9, s), 10.20(1HX0.1, s) |
| | | mp: 185-186 (EtOH) |
| | | F+: 500 |
| 18 | 4-F-3,5-(Me)₂-Ph | N1: 2.00-2.05(4H, m), 2.24(6H, s), 2.67-2.74(1H. m), 3.0 0-3.04(4H, m), 4.33(2H, S), 7.23(2H, d), 7.65 (2H, d), 7.74(2H, d), 8.43(1H, s), 8.54(1H, s), 10.15(1H, s) |
| | | mp: 188-189 (EtOH) |
| | | F+: 504 |
| 19 | 3,5-F₂-4-Me-Ph | N1: 1.99-2.05(4H, m), 2.17(3H, s), 2.75-2.82(1H, m), 2.9 9-3.10(4H, m), 4.37(2H, S), 7.28(2H, d), 7.65 (2H, d), 7.74(2H, d), 8.44(1H, s), 8.55(1H, s), 10.21(1H, s) |
| | | mp: 221-223 (EtOH) |
| | | F+: 486 |
| 20 | 2-F-4-Me-Ph | N1: 1.89-2.11(4H, m), 2.3.0(3HX0.1, s), 2.36(3HX0.9, s), 2.60-2.68(1H, m), 3.01-3.26(4H, m), 3.94(1HX0.9, d), 4. 02(1HX0.1, d), 4.50(1HX0.1, d), 4.76(1HX0.9, d), 7.00(1 HX0.1, d), 7.09(1HX0.1, d), 7.12(1HX0.9, d), 7.24(1HX0 .9, d), 7.38 (1HX0.1, dd), 7.50(1HX0.9, dd), 7.63(2H, d) , 7.73(2H, d), 8.44(1H, s), 8.55(1H, s), 10.17(1HX0.9, s ), 10.23(1HX0.1, s) |

**[Table 9]**

| | | |
|---|---|---|
| 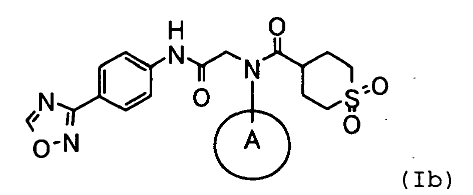 | | |

| **Ex** | **A** | **Dat** |
|---|---|---|
| | | F+: 469 |
| 21 | 4-Me-Ph | N1: 1.94-2.11(4H, m), 2.34(3H, s), 2.65-2.75(1H, m), 2.92-3.08(4H, m), 4.38(2H, s), 7.28(2H, d), 7.37(2H, d), 7.79(2 H, d), 8.00(2H, d), 9.66(1H, s), 10.38(1H, s) |
| | | mp: 203-205 (EtOH) |
| | | F-: 467 |
| 22 | 3-Me-Ph | N1: 1.96-2.11(4H, m), 2.35(3H, s), 2.65-2.76(1H, m), 2.92-3.09(4H, m), 4.39(2H, s), 7.20-7.39(4H, m), 7.79(2H, d), 8.00(2H, d), 9.66(1H, s), 10.38(1H, s) |
| | | F+: 469 |
| 23 | 2-Me-Ph | N1: 1.88-2.26(4H+3H, m), 2.42-2.52(1H, m), 2.84-3.18(4H, m), 3.91(1H × 0.9, d), 4.44(1H × 0.1, d), 4.75(1H × 0.9, d), 4.87(1H × 0.1, d), 7.08-7.54(4H, m), 7.75-7.81(2H. m), 7. 97-8.04(2H, m), 9.66(1H × 0.9, s), 9,67(1H × 0.1, s), 10.37 (1H × 0.9, s), 10.41(1H × 0.1, s) |
| | | mp: 216-217 (MeOH) |
| | | F-: 481 |
| 24 | 2,3-(Me)₂-Ph | N1: 1.83-2.31 (4H+3H+3H, m), 2.42-2.54(1H, m), 2.82-3.16( 4H, m), 3.88(1H × 0.9, d), 4.19(1H × 0.1, d), 4.72(1H × 0.9, d), 4.87(1H × 0.1, d), 7.05-7.37(3H, m), 7.75-7.80(2H, m) , 7.97-8.03(2H, m), 9.66(1H × 0.9, s), 9.66(1H × 0.1, s), 1 0.35(1H × 0.9, s), 10.38(1H × 0.1, s) |
| | | mp: 223-225 (EtOH) |
| | | F-: 481 |
| 25 | 2,4-(Me)₂-Ph | N1: 1.84-2.33(4H+3H+3H, m), 2.42-2.52(1H, m), 2.84-3.19( 4H, m), 3.86(1H × 0.9, d), 4.21(1H × 0.1, d), 4.73(1H × 0.9, d), 4.84(1H × 0.1, d), 6.95-7.40(3H, m), 7.75-7.81(2H, m) , 7.98-8.02(2H, m), 9.66(1H × 0.9, s), 9.66(1H × 0.1, s), 1 0.35(1H × 0.9, s), 10.39(1H × 0.1, s) |
| | | mp: 139-141 (EtOH) |

**[Table 10]**

| | | |
|---|---|---|
| | | F-: 481 |
| 26 | 2,5-(Me)₂-Ph | N1: 1.84-2.32(4H+3H+3H, m), 2.42-2.52(1H, m), 2.87-3.18( 4H, m), 3.89(1H × 0.9, d), 4.25(1H × 0.1, d), 4.72(1H × 0.9, d), 4.83(1H × 0.1, d), 6.92-7.34(3H, m), 7.76-7.82(2H, m) , 7.98-8.04(2H, m), 9.66(1H × 0.9, s), 9.67(1H × 0.1, s), 1 0.37(1H × 0.9, s), 10.39(1H × 0.1, s) |
| | | mp: 214-217 (EtOH) |
| | | F-: 481 |
| 27 | 2,6-(Me)₂-Ph | N1: 1.88-2.42(5H+6H, m), 2.98-3.27(4H, m), 4.22(2H × 0.86, s) 4.51(2H × 0.14, s), 7.1-7.3(3H, m), 7.76-7.81(2H, m), 7.99-8.03(2H, m), 9.66(1H, s), 10.38(1H, s) |
| | | mp:220-222(EtOH-H₂O) |
| | | F+: 483 |
| 28 | 3,4-(Me)₂-Ph | N1: 1.97-2.20(4H, m), 2.24(6H, s), 2.67-2.76(1H, m), 2.96-3.30(4H, m), 4.37(2H, s), 7.17-7.27(3H, m), 7.79(2H, d), 8.00(2H, d), 9.66(1H, s), 10.36(1H, s) |
| | | mp: 141-143 (EtOH) |
| | | F+: 483 |
| 29 | 3,5-(Me)₂-Ph | N1: 1.98-2.12(4H, m), 2.30(6H, s), 2.65-2.78(1H, m), 2.93-3.10(4H, m), 4.36(2H, s), 7.00-7.12(3H, m), 7.79(2H, d), 8.00(2H, d), 9.66(1H, s), 10.37(1H, s) |
| | | mp: 197-198 (iPrOH) |
| | | F-: 495 |
| 30 | 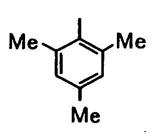 | N1: 1.83-2.52(4H+9H+1H, m), 2.99-3.26(4H, m), 4.18(2Hx 0.9, s), 4.48(2H × 0.1, s), 6.88(2H × 0.1, s), 7.01(2H × 0.9, s), 7.74-7.82(2H, m), 7.94-8.03(2H, m), 9.66(1H, s), 10. 36(1H, s) |
| | | mp: 188-190 (EtOH) |
| | | F-: 499 |
| 31 | 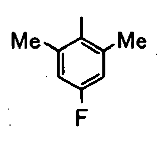 | N1: 1.82-2.44(6H+5H, m), 2.98-3.30(4H, m), 4.21(2H × 0.85, s), 4.50(2H × 0.15, s), 6.95(2H × 0.15, d), 7.08(2H × 0.85, d), 7.75-7.82(2H, m), 7.97-8.04(2H, m), 9.66(1H × 0.85, s ), 9.66(1H × 0.15, s), 10.40(1H, brs) |
| | | mp: 226-229 (EtOH-MeCN-H₂O) |

**[Table 11]**

| | | |
|---|---|---|
| | | F+: 487 |
| 32 | 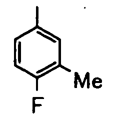 | N1: 1.97-2.11(4H, m), 2.26(3H, brs), 2.63-2.74(1H, m), 2. 95-3.07(4H, m), 4.38(2H, s), 7.21-7.45(3H, m), 7.79(2H, d), 8.00(2H, d), 9.66(1H, s), 10.39(1H, s) |
| | | mp: 136-138 (EtOH) |
| | | F-: 493 |
| 33 | 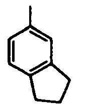 | N1: 1.96-2.20(6H, m), 2.70-2.78(1H, m), 2.84-3.08(8H, m) , 4.37(2H, s), 7.04-7.33(3H, m), 7.79(2H, d), 8.00(2H, d ), 9.66(1H, s), 10.37(1H, s) |
| | | F-: 546 |
| 34 | 4-Me-3-Br-Ph | N1: 1.96-2.16(4H, m), 2.38(3H, s), 2.66-2.77(1H, m), 2.9 6-3.08(4H, m), 4.39(2H, s), 7.40-7.49(2H, m), 7.73-7.82( 3H, m), 8.00(2H, d), 9.66(1H, s), 10.41(1H, s) |
| | | mp: 203-206 (iPrOH) |
| | | F+: 487 |
| 35 | 3-F-4-Me-Ph | N1: 1.97-2.07 (4H, m), 2.26 (3H, s), 2.69-2.77 (1H, m), 2.99-3.03 (4H, m), 4.39 (2H, s), 7.22-7.28 (1H, m), 7.31-7.42 (2H, m), 7.80 (2H, d), 7.99 (2H, d), 9.66 (1H, s), 10.40 (1H, s) mp: 200-203 (EtOH) |
| | | F+: 503 |
| 36 | 3-Cl-4-Me-Ph | N1: 1.97-2.11 (4H, m), 2.36 (3H, s), 2.65-2.78 (1H, m), 2 .97-3.08 (4H, m), 4.39 (2H, s), 7.39 (1H, dd), 7.45 (1H d), 7.60 (1H, d), 7.80 (2H, d), 7.99 (2H, d), 9.65 (1H, s), 10.40 (1H, s) |
| | | mp: 204-205 (EtOH) |
| | | F+: 503 |
| 37 | 4-Cl-3-Me-ph | N1: 1.95-2.09 (4H, m), 2.36 (3H, s), 2.65-2.76 (1H, m), 2 .95-3.07 (4H, m), 4.39 (2H, s), 7.36 (1H, dd), 7.48 (1H , d), 7.51 (1H, d), 7.80 (2H, d), 7.99 (2H, d), 9.66 (1H, s), 10.40 (1H, s) |
| | | mp: 196-199 (EtOH) |
| | | F+: 501 |
| 38 | 4-F-3,5-(Me)₂-Ph | N1: 1.94-2.12(4H, m), 2.24(6H, s), 2.64-2.74(1H, m), 2.9 4-3.08(4H, m), 4.35(2H, s), 7.23(2H, d), 7.79(2H, d), 7. 99(2H, d), 9.66(1H, s), 10.38(1H, s) |

**[Table 12]**

| | | |
|---|---|---|
| | | F+: 501 |
| 39 | 3-F-2,4-(Me)₂-Ph | N1: 1.84-2.34(4H+3H+3H, m), 2.48-2.55(1H, m), 2.85-3.22 (4H, m), 3.98(1H × 0.9, d), 4.30(1H × 0.1, d), 4.65(1H × 0 .9, d), 4.81(1H × 0.1, d), 7.22(1H, t), 7.27(1H, d), 7.78(2 H, d), 7.98(2H, d), 9.66(1H, s), 10.37(1H × 0.9, s), 10.5 1(1H × 0.1, s) |
| | | F+: 487 |
| 40 | 2-F-4-Me-Ph | N1: 1.90-2.18(4H, m), 2.30(3HX0.1, s), 2.36(3HX0.9, s), 2.62-2.68(1H, m), 3.01-3.23(4H, m), 3.99(1H, d), 4.77(1 H, d), 7.13(1H, d), 7.25(1H, d), 7.50(1H, dd), 7.77(2H, d), 7.99(2H, d), 9.66(1H, s), 10.40(1HX0.9, s), 10.45(1H X0.1, s) |
| | | mp: 197-198 (EtOH) |

### Industrial Applicability

By separate administration of a polymerase inhibitor acyclovir, valacyclovir or famciclovir as a conventional anti-herpesvirus agent with a helicase-primase inhibitor of general formula (I) having a different functional mechanism, the anti-herpesvirus agent of the present invention achieved an anti-herpesvirus activity. Therefore, it is particularly effective in a case in which a sufficient therapeutic effect cannot be achieved only with a polymerase inhibitor. In addition, since the doses of both agents can be kept low by their separate use, it is possible to obtain a treatment effect which is similar to or greater than the case of respective single administration, together with lowering the undesirable actions of both agents to be concerned. Accordingly, the anti-herpesvirus agent of the present invention is useful as an anti-herpesvirus agent particularly having high safety for the prevention or treatment of various herpesvirus infections such as varicella (chickenpox) associated with VZV infection, herpes zoster associated with recurrent infection with latent VZV, labial herpes and herpes encephalitis associated with HSV-1 infection, genital herpes associated with HSV-2 infection.

## Claims

1. An anti-herpesvirus agent **characterised by** combining a compound of the general formula (I): 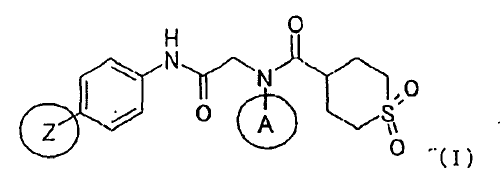 wherein Z is 1,2,4-oxadiazol-3-yl or 4-oxazolyl; A is a phenyl group which is substituted by a at least one methyl group and may have 1 or 2 further substituents selected from the group consisting of a methyl group and halogen atoms, or a 5-indanyl group; with a polymerase inhibitor selected from acyclovir, valacyclovir and famciclovir; wherein the compound of general formula (I) and the polymerase inhibitor are to be administered separately.

2. An anti-virus agent according to claim 1 wherein the polymerase inhibitor is valacyclovir.

## Patentansprüche

1. Anti-Herpesvirus-Mittel, **gekennzeichnet durch** Kombinieren einer Verbindung der allgemeinen Formel (I): 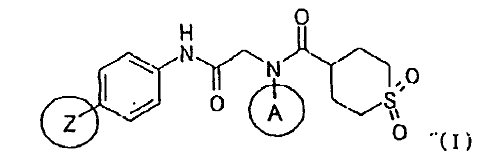 wobei Z 1,2,4-Oxadiazol-3-yl oder 4-Oxazolyl ist; A eine Phenylgruppe ist, die substituiert ist **durch** wenigstens eine Methylgruppe und 1 oder 2 weitere Substituenten haben kann, die ausgewählt sind aus der Gruppe bestehend aus einer Methylgruppe und Halogenatomen oder einer 5-Indanylgruppe; mit einem Polymeraseinhibitor, der ausgewählt ist aus Acyclovir, Valacyclovir und Famciclovir; wobei die Verbindung der allgemeinen Formel (I) und der Polymeraseinhibitor getrennt zu verabreichen sind.

2. Antivirusmittel nach Anspruch 1, wobei der Polymeraseinhibitor Valacyclovir ist.

## Revendications

1. Agent contre un virus de l'herpès **caractérisé en** combinant un composé de la formule générale (I) : 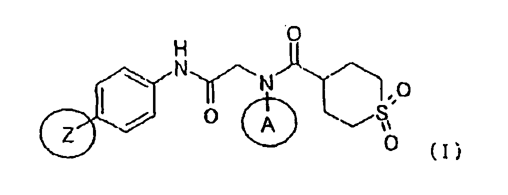 où Z est un 1,2,4-oxadiazol-3-yle ou un 4-oxazolyle; A est un groupe phényle qui est substitué par au moins un groupe méthyle et peut avoir 1 ou 2 substituants supplémentaires sélectionnés parmi le groupe consistant en un groupe méthyle et des atomes d'halogène, ou bien un groupe 5-indanyle; avec un inhibiteur de polymérase sélectionné parmi acyclovir, valacyclovir et famciclovir; où le composé de la formule générale (I) et l'inhibiteur de polymérase doivent être administrés séparément.

2. Agent antiviral selon la revendication 1, dans lequel l'inhibiteur de polymérase est le valacyclovir.
